# EUROPÄISCHE PATENTSCHRIFT

(11) **EP 1 616 590 B1**
(45) Veröffentlichungstag und Bekanntmachung des Hinweises auf die Patenterteilung: **31.03.2010**
(21) Anmeldenummer: 04016844.5
(22) Anmeldetag: 16.07.2004
(51) Int. Cl.: A61M 5/19, A61M 5/28, A61C 9/00

(54) **Mehrkammerampulle zum Ausgeben eines aus mehreren Substanzen bestehenden Gemisches**
Multichamber ampoule for dispensing a mixture consisting of multiple substances
Ampoule multi-chambre pour la distribution d'un mélange de plusieurs substances

(43) Veröffentlichungstag der Anmeldung: 18.01.2006
(73) Patentinhaber: Dentaco Dentalindustrie und -marketing GmbH, 61352 Bad Homburg (DE)
(72) Erfinder: Sogaro, Alberto C., 61476 Kronberg (DE)
(74) Vertreter: advotec.

(56) Entgegenhaltungen:
- EP-A- 1 426 017
- DE-U- 20 107 507

## Beschreibung

Die Erfindung betrifft eine Mehrkammerampulle zum Ausgeben eines aus mehreren Substanzen bestehenden Gemisches gemäss dem Oberbegriff des Patentanspruchs 1. Eine derartige Mehrkammerampulle ist aus der EP 1 203 593 A1 bekannt. Diese bekannte Mehrkammerampulle war zum einmaligen Gebrauch als Wegwerfartikel konzipiert.

Eine Mehrkammerampulle nach dem Oberbegriff des Patentanspruchs 1 ist auch aus der EP 1 426 017 A2 bekannt.

Aufgabe der Erfindung ist es, eine zum mehrmaligen Gebrauch geeignete Mehrkammerampulle zu schaffen, die dennoch einfach ausgebildet, leicht zu handhaben und kostengünstig herzustellen ist.

Diese Aufgabe wird durch den Gegenstand des Anspruchs 1 gelöst. Bevorzugte Weiterbildungen der Erfindung sind in Unteransprüchen definiert.

Die Erfindung macht es möglich, dass unmittelbar nach Beendigung einer Gebrauchsanwendung der Mehrkammerampulle der Adapter zusammen mit dem Mehrstopfenverschluss vom Behälter der Mehrkammerampulle getrennt und durch eine neue Adapter-Mehrstopfenverschluss-Einheit ersetzt werden kann. Die Erfindung bietet den Vorteil, dass nicht nur der Adapter, sondern gleichzeitig auch der Mehrstopfenverschluss ohne zusätzliche Massnahmen vom Behälter getrennt und entsorgt werden können. Mit der Entfernung des Mehrstopfenverschlusses werden auch daran anhaftende bereits aus den Kammern ausgetretene Anteile der im Übrigen in den Kammern aufbewahrten Substanzen beseitigt. Die neue eingesetzte Adapter-Mehrstopfenverschluss-Einheit verschließt dann in den Kammern ausschließlich noch nicht miteinander in Berührung gebrachte Anteile der Substanzen.

Die erfindungsgemäße Lösung besteht im Prinzip darin, für eine zum mehrfachen Gebrauch gedachte Mehrkammerampulle eine aus einem Adapter und einem Mehrstopfenverschluss bestehende Einheit vorzusehen, bei der der Mehrstopfenvershluss am Adapter drehbeweglich, jedoch im übrigen im wesentlichen nicht verschieblich montiert ist, so dass lediglich durch Manipulation des Adapters die genannte Einheit in den Behälter eingesetzt, aus dem Behälter herausgenommen und im eingesetzten Zustand durch Verschieben und/oder Drehen des Adapters in gewünschte Betriebszustände gebracht werden kann.

Gemäss einer bevorzugten Weiterbildung der Erfindung sind am Adapter und am Mehrstopfenverschluss Rastmittel angeformt, die derart zusammenwirken, dass der Adapter und der Mehrstopfenverschluss in einer vorgegebenen Drehwinkelstellung miteinander verrastbar sind. Dies hat den Vorteil, dass beim anfänglichen Einsetzen der genannten Einheit in den Behälter lediglich der Adapter mit dem Behälter drehwinkelmässig auszurichten ist und der Mehrstopfenverschluss automatisch eine solche Drehwinkelstellung einnimmt, dass die Stopfen des Mehrstopfenverschlusses mit den Austrittsöffnungen der Kammern ausgerichtet sind.

Gemäss einer anderen Weiterbildung der Erfindung ist seitlich an den Mehrstopfenverschluss ein in Radial- oder Querrichtung vorspringender Ansatz angeformt und am Behälter ist eine Aussparung ausgebildet. Die Aussparung wirkt mit dem Ansatz derart zusammen, dass der Mehrstopfenverschluss nur in einer vorgegebenen Drehwinkelstellung in den Behälter eingesetzt werden kann, in der die Stopfen mit den Austrittsöffnungen der Kammern ausgerichtet sind. Auch dies erleichtert das anfängliche Einsetzen der genannten Einheit, insbesondere in Kombination mit der oben erläuterten drehwinkelmässigen Verrastung von Adapter und Mehrstopfenverschluss.

Die oben erläuterte drehwinkelmässige Ausrichtung von Mehrstopfenverschluss und Behälter erleichtert das anfängliche Einsetzen insbesondere dann, wenn die Austrittsöffnungen der Kammern und damit auch die dazu passenden Stopfen voneinander abweichende Querschnittsabmessungen haben.

Ist im Ausgabekanal des Adapters ein statischer Mischer angeordnet, wird beim Ersetzen der Adapter-Mehrstopfenverschluss-Einheit selbstverständlich auch der Mischer ausgetauscht.

Bevorzugte Ausführungsbeispiele der Erfindung werden nachstehend anhand von Zeichnungen erläutert, wobei zur besseren Veranschaulichung in einigen der Zeichnungen nicht sichtbare Kanten gestrichelt dargestellt sind. Es zeigen:
Fig. 1 eine teilweise geschnittene Seitenansicht eines ersten Ausführungsbeispiels einer nach der Erfindung ausgebildeten Mehrkammerampulle im verschlossenen Zustand,
Fig. 2 eine unter dem gleichen Blickwinkel wie in Fig. 1 dargestellte, teilweise geschnittene Seitenansicht der Mehrkammerampulle im aktivierten Zustand,
Fig. 3 eine unter dem gleichen Blickwinkel wie in Fig. 1 und 2 dargestellte, teilweise geschnittene Seitenansicht der Mehrkammerampulle im entleerten Zustand,
Fig. 4 eine geschnittene Seitenansicht eines Behälters der Mehrkammerampulle,
Fig. 5 eine gegenüber Fig. 4 im Blickwinkel um 90° gedrehte Seitenansicht des Behälters,
Fig. 6 eine Draufsicht des in Fig. 4 und 5 dargestellten Behälters,
Fig. 7 eine Seitenansicht einer Kolbeneiriheit des Mehrkammerampulle,
Fig. 8 eine Draufsicht der in Fig. 7 dargestellten Kolbeneinheit,
Fig. 9 eine geschnittene Seitenansicht eines Adapters der Mehrkammerampulle,
Fig. 10 eine Draufsicht des in Fig. 9 dargestellten Adapters,
Fig. 11 eine gegenüber Fig. 9 im Blickwinkel um 90° gedrehte Seitenansicht des Adapters,
Fig. 12 eine Draufsicht des in Fig. 11 dargestellten Adapters,
Fig. 13 eine Seitenansicht eines Mehrstopfenverschlusses der Mehrkammerampulle in einem vergrösserten Massstab,
Fig. 14 eine Draufsicht des in Fig. 13 dargestellten Mehrstopfenverschlusses,
Fig. 15 eine geschnittene Seitenansicht des in Fig. 13 und 14 dargestellten Mehrstopfenverschlusses,
Fig. 16 eine Seitenansicht eines Behälters eines zweiten Ausführungsbeispiels einer nach der Erfindung ausgebildeten Mehrkammerampulle,
Fig. 17 eine gegenüber Fig. 16 im Blickwinkel um 90° gedrehte Seitenansicht des Behälters,
Fig. 18 eine Draufsicht des in Fig. 16 und 17 dargestellten Behälters,
Fig. 19 eine geschnittene Seitenansicht eines Adapters des zweiten Ausführungsbeispiels der Mehrkammerampulle,
Fig. 20 eine gegenüber Fig. 19 im Blickwinkel um 90° gedrehte Seitenansicht des Adapters,
Fig. 21 eine Seitenansicht eines Mehrstopfenverschlusses des zweiten Ausführungsbeispiels der Mehrkammerampulle,
Fig. 22 eine Draufsicht des in Fig. 21 dargestellten Mehrstopfenverschlusses,
Fig. 23 eine geschnittene Seitenansicht zur Erläuterung einer drehbeweglichen Verbindung zwischen Adapter und Mehrstopfenverschluss,
Fig. 24 eine Seitenansicht eines Teils des hinteren Abschnitts des in Fig. 19 dargestellten Adapters und eine geschnittene Sietenansicht eines auf den hinteren Adapterabschnitt aufschiebbaren Anschlagstücks in Form eines Clip,
Fig. 25 eine Ansicht von oben auf den in Fig. 24 dargestellten Clip,
Fig. 26 eine teilweise geschnittene Seitenansicht des Adapters und Behälters einer ersten Weiterbildung des ersten Ausführungsbeispiels im verschlossenen Zustand,
Fig. 27 eine Draufsicht des in Fig. 26 dargestellten Behälters,
Fig. 28 eine teilweise geschnittene Seitenansicht des in Fig. 26 dargestellten Adapters und Behälters der ersten Weiterbildung des ersten Ausführungsbeispiels im aktivierten Zustand,
Fig. 29 eine Draufsicht des in Fig. 28 dargestellten Behälters,
Fig. 30 eine teilweise geschnittene Seitenansicht des Adapters und Behälters einer zweiten Weiterbildung des ersten Ausführungsbeispiels im verschlossenen Zustand,
Fig. 31 eine Draufsicht des in Fig. 26 dargestellten Behälters,
Fig. 32 eine teilweise geschnittene Seitenansicht des in Fig. 26 dargestellten Adapters und Behälters der zweiten Weiterbildung des ersten Ausführungsbeispiels im aktivierten Zustand, und
Fig. 33 eine Draufsicht des in Fig. 32 dargestellten Behälters.

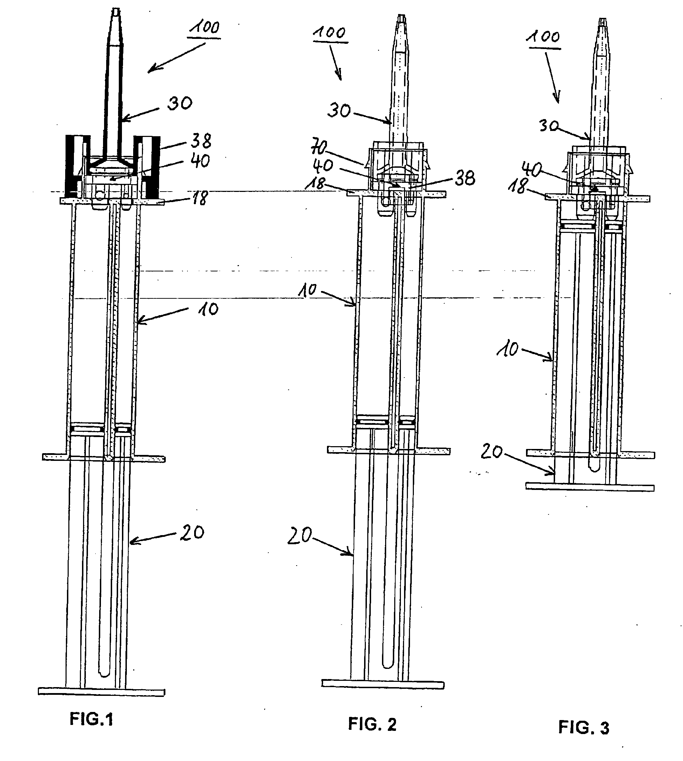

Ein in Fig. 1 bis 15 dargestelltes erstes Ausführungsbeispiel der Erfindung ist in Form einer Zweikammerampulle 100 ausgebildet. Die erfindungsgemässe Zweikammerampulle 100 besteht im wesentlichen aus vier Bauteilen, nämlich einem Behälter 10, einer Kolbeneinheit 20, einem Adapter 30 und einem Mehrstopfenverschluss 40.

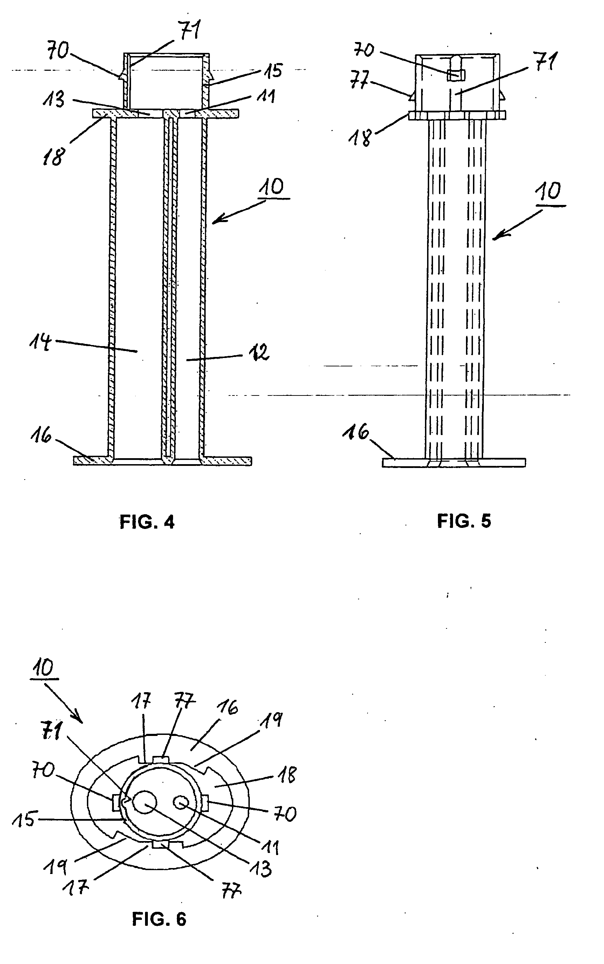

Wie es insbesondere aus Fig. 4 bis 6 hervorgeht, hat der Behälter 10 zwei parallel nebeneinander angeordnete, rohrförmige Kammern 12 und 14, die sich in Längsrichtung des Behälters 10 erstrecken. Die Kammern 12 und 14 sind an ihrem hinteren Ende über ihren gesamten Querschnitt offen. An die Aussenseiten der hinteren Enden der Kammern 12 und 14 ist eine sich in Quer- oder Radialrichtung erstreckende Rückplatte 16 angeformt. Die vorderen Enden der Kammern 12 und 14 sind über eine angeformte Frontplatte 18 miteinander verbunden. In der Frontplatte 18 sind eine Austrittsöffnung 11 für die Kammer 12 und eine Austrittsöffnung 13 für die Kammer 14 ausgebildet. Während die Kammern 12 und 14 an die Unterseite der Frontplatte 18 angrenzen, ist an die Oberseite der Frontplatte 18 ein Hals 15 in einer solchen Weise angeformt, dass der Hals 15 die Austrittsöffnungen 11 und 13 umrahmt. Der Hals 15 erstreckt sich ebenso wie die Kammern 12 und 14 in Längsrichtung des Behälters 10. Bei dem betrachteten Ausführungsbeispiel hat die Kammer 14 einen viermal grösseren Querschnitt als die Kammer 12. Dementsprechend hat auch die Austrittsöffnung 13 einen viermal grösseren Querschnitt als die Austrittsöffnung 11.

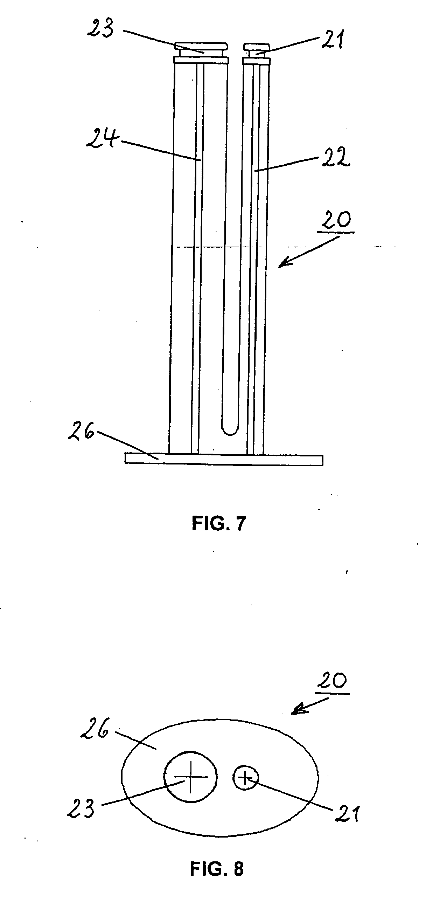

Wie es insbesondere aus Fig. 7 und 8 hervorgeht, besteht die Kolbeneinheit 20 aus zwei Kolbenstangen 22 und 24, deren hintere Enden über eine Drückerplatte 26 miteinander verbunden sind. Am vorderen Ende der Kolbenstange 22 ist ein Kolben 21 für die Kammer 12 angebracht. Am vorderen Ende der Kolbenstange 24 ist ein Kolben 23 für die Kammer 14 angebracht. Die Kolben 21 und 23 haben äussere Querschnittsabmessungen, die den inneren Querschnittsabmessungen der Kammern 12 und 14 entsprechen. Die Kolben 21 und 23 können in die hinteren offenen Enden der Kammern 12 und 24 eingesetzt und in den Kammern 12 und 14 fluiddicht verschoben werden

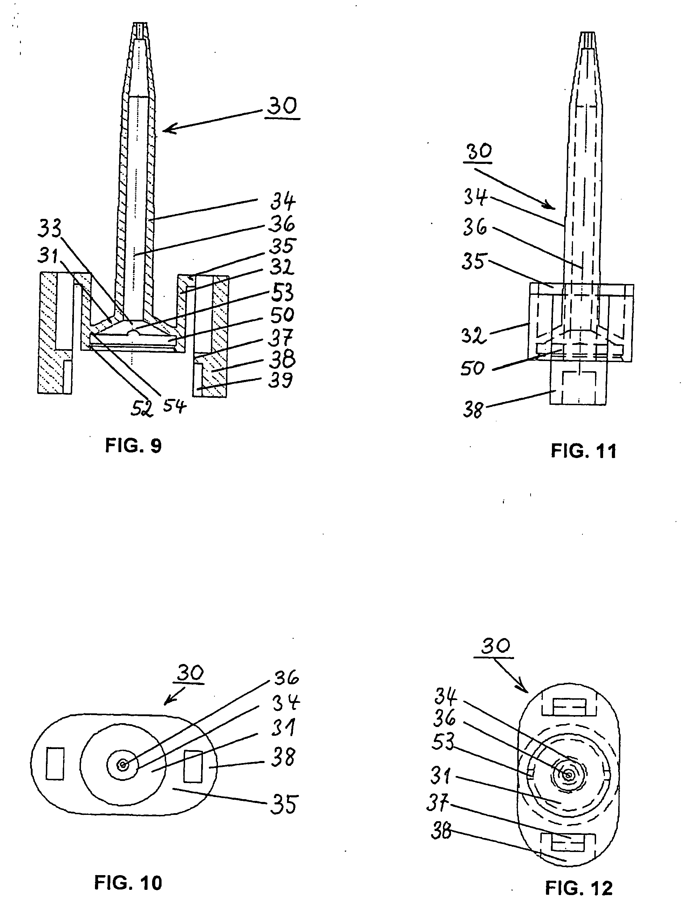

Wie es insbesondere aus Fig. 9 bis 12 hervorgeht, hat der in Form eines Ausgabestücks ausgebildete Adapter 30 einen zylindrischen hinteren Abschnitt 32. Der zylindrische Abschnitt 32 hat einen solchen Aussendurchmesser, dass der hintere Abschnitt 32 von oben her in das offene vordere Ende des Halses 15 einsetzbar und darin fluiddicht verschiebbar und drehbar ist. In einem Abstand vom hinteren Ende ist an die Innenfläche des zylindrischen Abschnitts 32 eine schräg nach innen und vorne gerichtete Umfangswand 31 angeformt. Die Umfangswand 31 begrenzt einen nach hinten offenen, kegelstumpfförmigen Mischraum 33. Das engere vordere Ende der Umfangswand 31 geht in ein rohrförmiges Gebilde über, das das vordere Ende des hinteren Abschnitts 32 überragt und einen vorderen Abschnitt 34 des Adapters 30 dargestellt. Innerhalb des vorderen Abschnitts 34 erstreckt sich in Längsrichtung des Adapters 30 ein Ausgabekanal 36. Das hintere Ende des Ausgabekanals 36 und das vordere Ende des Mischraums 33 gehen ineinander über.

An das vordere Ende des zylindrischen Abschnitts 32 ist ein radial nach aussen ragender Querträger 35 angeformt. An die Unterseite des Querträgers 35 sind in einem Abstand vom Aussenumfang des zylindrischen Abschnitts 32 nach hinten ragende Verriegelungsarme 38 angeformt.

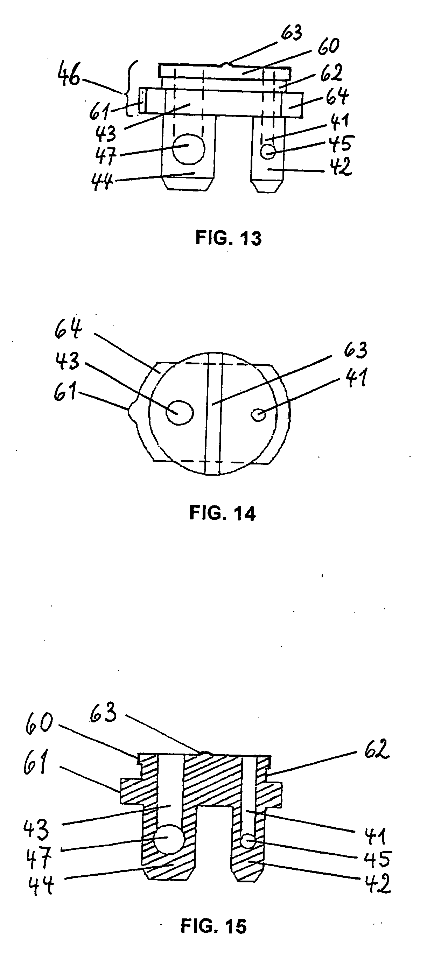

Wie es insbesondere aus Fig. 13 bis 15 hervorgeht, hat der Mehrstopfenverschluss 40 zwei Stopfen 42 und 44, die an die Unterseite eines gemeinsamen plattenförmigen Körpers 46 angeformt sind. Der plattenförmige Körper 46 besteht angrenzend an die Stopfen 42 und 44 aus einer Grundplatte 64, einer an die Oberseite der Grundplatte angeformten Zwischenplatte 62 und einer an die Oberseite der Zwischenplatte angeformten Kopfplatte 60, die das vordere Ende des Mehrstopfenverschlusses 40 darstellt.

Von der Oberseite der Kopfplatte 60 aus erstrecken sich zwei Längskanäle 41 und 43 nach unten bis in die Stopfen 42 und 44. Der Längskanal 41 mündet in einen den Stopfen 42 in Querrichtung durchsetzenden Querkanal 45. Der Längskanal 43 mündet in einen den Stopfen 44 querdurchsetzenden Querkanal 47. Die Querkanäle 45 und 47 sind in einem vorgegebenen Abstand von den unteren Enden der Stopfen 42 und 44 ausgebildet.

Die Kopfplatte 60 hat eine kreisrunde Umfangswand mit einem Durchmesser, der grösser als derjenige der Zwischenplatte 62 ist. Zum Herstellen einer Drehverbindung zwischen dem Adapter 30 und dem Mehrstopfenverschluss 40 kann die Kopfplatte 60 vom hinteren Ende des Adapters 30 her in eine ringförmige Halterungsnut 50 eingeschnappt werden, die unterhalb der Umfangswand 31 in der Innenfläche des zylindrischen Abschnitts 32 des Adapters 30 ausgebildet ist. Die Nut 50 und die Kopfplatte 60 sind bezüglich ihrer Abmessungen derart aufeinander abgestimmt, dass die Kopfplatte 60 in der Nut 50 drehbeweglich gehaltert ist. Eine Herausnahme der Kopfplatte 60 aus der Nut 50 ist durch eine ringförmige Schulter 52 verhindert, die am hinteren Ende der Innenfläche des zylindrischen Abschnitts 32 ausgebildet ist. Damit bei der Montage die Kopfplatte 60 leichter in der Nut 50 eingeschnappt werden kann, ist die radial nach innen weisende Fläche der Schulter 52 in der gezeigten Weise abgeschrägt. Eine Längsverschiebung der Kopfplatte 60 nach oben oder vorne ist durch eine Schulter 54 begrenzt, die an der Unterseite der Umfangswand 31 ausgebildet ist.

Auf der Oberseite der Kopfplatte 60 ist eine Querrippe 63 angeformt, die mit zwei diametral einander gegenüberliegenden Aussparungen 53 in der Schulter 54 derart zusammen wirkt, dass der im Adapter 30 drehbar gehalterte Mehrstopfenverschluss 40 in einer vorgegebenen Winkel- oder Drehstellung lösbar verrastet werden kann.

Der Adapter 30 und der Mehrstopfenverschluss 40 sind somit derart ausgestaltet, dass diese beiden Bauteile eine einzige Baueinheit 30, 40 bilden.

Beim Einsetzen der aus Adapter 30 und Mehrstopfenverschluss 40 bestehenden Baueinheit von vorne oder von oben her in den Hals 15 des Behälters 10 treten die Stopfen 42 und 44 in die Austrittsöffnungen 11 und 13 der Kammern 12 und 14 ein. Die Stopfen 42 und 44 sind im Vergleich zu den Austrittsöffnungen 11 und 13 derart dimensioniert, dass die Stopfen in einer ersten Eintrittsstellung mit ihren hinteren Endabschnitten die Austrittsöffnungen 11 und 13 fluiddicht verschliessen. Hierbei befinden sich die Querkanäle 45 und 47 oberhalb der Frontplatte 18. Der in die Austrittsöffnung 13 eingetretene hintere Abschnitt des Stopfens 44 hat einen viermal grösseren Querschnitt als der in die Austrittsöffnung 11 eingetretene hintere Abschnitt des Stopfens 42.

In einer weiter nach unten oder hinten verschobenen zweiten Eintrittsstellung der Stopfen 42 und 44 befinden sich die Querkanäle 45 und 47 unterhalb der Frontplatte 18, so dass über die Querkanäle 45 und 47 sowie die Längskanäle 41 und 43 eine Fluidverbindung zwischen dem Inneren der Kammern 12 und 14 und dem Mischraum 33 beziehungsweise dem Ausgabekanal 36 des Adapters 30 besteht. Der Innenquerschnitt des Längskanals 43 ist viermal grösser als derjenige des Längskanals 41. Entsprechendes gilt für den Querkanal 47 im Vergleich zum Querkanal 45.

Die Grundplatte 64 hat zur besseren Führung des Mehrstopfenverschlusses 40 im Hals 15 eine Umfangswand, die zumindest über Teilbereiche des Umfangs der Innenumfangswand des Halses 15 angepasst ist. In einem solchen Teilbereich der Umfangswand der Grundplatte 64 ist ein radial vorspringender Ansatz 61 angeformt. Der Ansatz 61 arbeitet beim anfänglichen Einsetzen des Mehrstopfenverschlusses 40 in den Hals 15 mit einer in der Innenfläche des Halses 15 ausgebildeten Längsnut 71 derart zusammen, dass der Mehrstopfenverschluss 40 nur in einer vorgegebenen Winkel- oder Drehstellung in den Hals 15 eingesetzt werden kann, in der die Stopfen 42 und 44 mit den Austrittsöffnungen 11 und 13 der Kammern 12 und 14 ausgerichtet sind.

An der Aussenumfangswand des Halses 15 sind in einem vorgegebenen Abstand von der Oberseite der Frontplatte 18 radial nach aussen ragende Verriegelungsnasen 70 angeformt. Ferner sind an der äusseren Umfangswand des Halses 15 weitere radial nach aussen ragende Verriegelungsnasen 77 angeformt, die gegenüber den Verriegelungsnasen 70 in Umfangsrichtung um 90° versetzt sind und einen geringeren Abstand von der Frontplatte 18 haben. Die Verriegelungsnasen 77 befinden sich über einem Umfangsbereich der Frontplatte 18, in dem in der Frontplatte 18 Aussparungen 17 ausgebildet sind. Die Verriegelungsnasen 77 ragen in Radialrichtung bei den Aussparungen 17 über die Aussenkante der Frontplatte 18 hinaus, wohingegen die Verriegelungsnasen 70 in einem nicht ausgesparten Umfangsbereich der Frontplatte 18 vorgesehen sind, in dem die Frontplatte 18 in Radialrichtung über die Verriegelungsnasen 70 hinausragt.

Auf der Innenseite der Verriegelungsarme 38 sind radial nach innen gerichtete Verriegelungsstege 37 und in Längsrichtung verlaufende Führungsnuten 39 ausgebildet, die angrenzend an die Unterseite der Verriegelungsstege 37 bis zum unteren Ende der Verriegelungsarme laufen. Die Verriegelungsstege 37 und die Führungsnuten 39 arbeiten mit den Verriegelungsnasen 70 und 77 zusammen. Die Stirnfläche des unteren Endes der Verriegelungsarme 38 arbeitet mit der Frontplatte 18 zusammen.

Diese Zusammenarbeit sowie der Zusammenbau und der Betrieb der Zweikammerampulle 100 wird im Folgenden insbesondere auch unter Bezugnahme auf Fig. 1 bis 3 erläutert.

Vor dem Anbringen der aus Adapter 30 und Mehrstopfenverschluss 40 bestehenden Baueinheit am Behälter 10 werden der Adapter 30 und der gegenüber dem Adapter drehbare Mehrstopfenverschluss 40 in einer vorgegebenen Drehstellung miteinander verrastet, in der der Raststeg 63 in die Rastausnehmungen 53 eingreift. In diesem verrasteten Zustand wird die Baueinheit 30, 40 von oben her in den Hals 15 des Behälters 10 eingeschoben. Hierbei ist der Ansatz 61 am Mehrstopfenverschluss 40 mit der Längsnut 71 im Hals 15 ausgerichtet. In dieser anfänglichen Drehstellung der Baueinheit 30, 40 gegenüber dem Behälter 10 sind die Verriegelungsarme 38 mit den Verriegelungsnasen 70 ausgerichtet. Beim Einschieben der Baueinheit 30, 40 in der anfänglichen Drehstellung treten deshalb die Verriegelungsnasen 70 in die Führungsnuten 39 ein, und die Stopfen 42 und 44 treten in die Austrittsöffnungen 11 und 13 der Kammern 12 und 14 ein. Wird in dieser ersten Drehstellung der Adapter 30 weiter in Richtung auf den Behälter 10 gedrückt, schnappen die Verriegelungsstege 37 über die Verriegelungsnasen 70. Damit wird eine Rückverschiebung der Baueinheit 30, 40 nach oben oder vorne aus dem Hals 15 des Behälters 10 heraus blockiert. Gleichzeitig wird in diesem blockierten Zustand eine Weiterverschiebung der Baueinheit 30, 40 in Richtung auf den Behälter 10 dadurch begrenzt, dass die Stirnflächen am unteren Ende der Verriegelungsarme 38 an der Oberseite der Frontplatte 18 anschlagen. Hierbei besteht zwischen dem plattenförmigen Körper 46 und der Frontplatte 18 ein vorgegebener Abstand und die Stopfen 42 und 44 sind gerade so weit in die Austrittsöffnungen 11 und 13 eingetreten, dass die Stopfen die Austrittsöffnungen fluiddicht verschliessen.

In der Verschlussstellung der Stopfen 42 und 44 können die Kammern 12 und 14 von ihrem offenen hinteren Ende her mit Substanzen gefüllt werden. Im Anschluss an die Befüllung mit den Substanzen werden die Kammern 12 und 14 von hinter her mit den Kolben 21 und 23 der Kolbeneinheit 20 verschlossen.

Diese Verschlussstellung durch die Stopfen und durch die Kolben ist in Fig. 1 dargestellt. Die in die Kammern 12 und 14 eingefüllten Substanzen sind nicht gezeigt.

Zum Aktivieren der mit den Substanzen gefüllten Zweikammerampulle 10 wird der Adapter 30 um 90° gegenüber dem Behälter 10 in eine zweite Drehstellung gedreht. Während der anfänglichen Drehung in die zweite Drehstellung werden die Verriegelungsarme 38 von den Verriegelungsnasen 70 seitwärts weggedreht, und gegen Ende der Drehung springen die Verriegelungsnasen 77 seitwärts in die Führungsnuten 39. Gleichzeitig gelangen die Verriegelungsarme 38 in Ausrichtung mit den Aussparungen 17 in der Frontplatte 18. Die Aussparungen 17 in der Frontplatte 18 ermöglichen es, dass die Baueinheit 30, 40 weiter in Richtung auf den Behälter 10 geschoben werden kann. Im Zuge dieser Verschiebung gelangen die Stopfen 42 und 44 in eine Durchlassstellung, in der sich die Querkanäle 45 und 47 im Inneren der Kammern 12 und 14 befinden. In der Durchlassstellung der Stopfen ragen die Verriegelungsarme 38 nach unten über die Frontplatte 18 hinaus und die Verriegelungsstege 37 schnappen über die Verriegelungsnasen 77. Die Verriegelungsstege 37 und die Verriegelungsnasen 77 verhindern, dass in der Durchlassstellung der Stopfen die Baueinheit 30, 40 nach vorne oder oben vom Behälter 10 weg gedrückt werden kann. Eine Weiterverschiebung der Baueinheit 30, 40 nach hinten oder unten wird dadurch verhindert, dass das obere Ende des Halses 15 an der Unterseite des Querträgers 35 anschlägt. Alternativ kann in der Durchlassstellung eine Weiterverschiebung der Baueinheit 30, 40 auch dadurch vermieden werden, dass der plattenförmige Körper 46 an der Frontplatte 18 anschlägt. Dieser aktivierte Zustand der Zweikammerampulle 100 ist in Fig. 2 dargestellt.

Durch Ausüben von Druck auf die Drückerplatte 26 der Kolbeneinheit 20 können jetzt die in den Kammern 12 und 14 enthaltenen Substanzen durch die Quer- und Längskanäle des Mehrstopfenverschlusses 40 in die Mischkammer 33 und den Ausgabekanal 36 gedrückt und durch die Spitze des vorderen Abschnitts 34 des Adapters 30 ausgegeben werden. Zum besseren Durchmischen der Substanzen ist im Ausgabekanal ein statischer Mischer vorgesehen, der nicht gezeigt ist.

Die dargestellte Zweikammerampulle 100 ist zum mehrmaligen Gebrauch gedacht. Es wird deshalb bei einer Anwendung lediglich ein Bruchteil der Substanzen in den Kammern 12 und 14 ausgebracht. Nach Beendigung einer Anwendung wird der Adapter 30 gegenüber dem Behälter 10 aus der zweiten Drehstellung um 45° in eine dritte Drehstellung gedreht. Dies wird dadurch ermöglicht, dass sich an die Aussparung 17 eine weitere Aussparung 19 anschliesst, die die Drehung des Adapters von der zweiten in die dritte Drehstellung ermöglicht. Bei der Drehung des Adapters in die dritte Drehstellung springen die Verriegelungsarme 38 seitlich aus den Verriegelungsnasen 77 heraus. Dadurch ist es in der dritten Drehstellung möglich, die Baueinheit 30, 40 aus dem Behälter 10 heraus nach oben abzuziehen. Jetzt kann, wie oben beschrieben, eine neue Baueinheit 30, 40 in der anfänglichen ersten Drehstellung am Behälter 10 angebracht werden.

Der Austausch der Baueinheit 30, 40 durch jeweils eine neue Baueinheit kann mehrmals vorgenommen werden, bis die Kammern 12 und 14 völlig entleert sind. Der entleerte Zustand der Mehrkammerampulle 10 ist in Fig. 3 dargestellt, wobei sich die Baueinheit 30, 40 in der zweiten Drehstellung befindet.

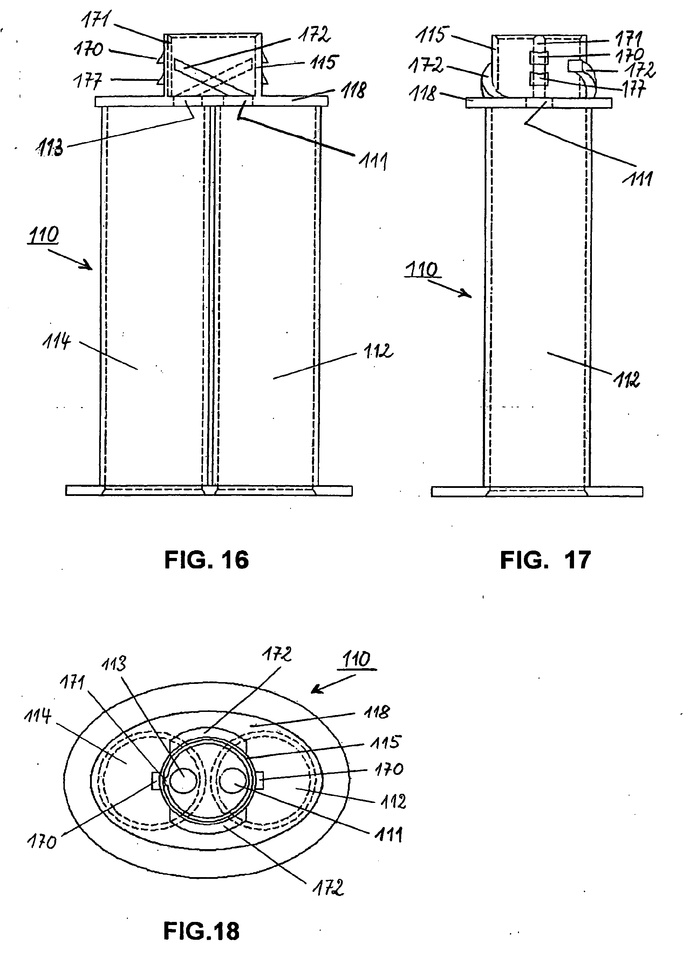

Ein in Fig. 16 bis 22 dargestelltes zweites Ausführungsbeispiel der Erfindung ist ebenfalls in Form einer Zweikammerampulle ausgebildet. Dementsprechend hat ein in Fig. 16 bis 18 dargestellter Behälter 110 des zweiten Ausführungsbeispiels zwei parallel nebeneinander angeordnete Kammern 112 und 114 mit in einer Frontplatte 118 ausgebildeten Austrittsöffnungen 111 und 113. Im Gegensatz zum Behälter 10 des ersten Ausführungsbeispiels haben allerdings die Kammern 112 und 114 einen gleichen Querschnitt. Entsprechendes gilt für die Austrittsöffnungen 111 und 113.

Wie beim Behälter 10 ist an die Oberseite der Frontplatte 118 ein Hals 115 angeformt, in den die Austrittsöffnungen 111 und 113 münden. Im Gegensatz zum Behälter 10 des ersten Ausführungsbeispiels sind jedoch die Aussenseite des Halses 115 und die Frontplatte 118 des Behälters 110 des zweiten Ausführungsbeispiels anders ausgestaltet. Im übrigen stimmt der Behälter 110 grundsätzlich mit dem Behälter 10 überein.

Wie es aus den Figuren 16 bis 18 hervorgeht, sind an der Aussenumfangswand des Halses 115 zwei radial nach aussen vorspringende Verriegelungsnasen 170 sowie zwei weitere radial nach aussen vorspringende Verriegelungsnasen 177 angeformt. Die Verriegelungsnasen 170 sind in einem vorgegebenen Abstand von der Oberseite der Frontplatte 118 vorgesehen und liegen diametral einander gegenüber. Die Verriegelungsnasen 177 sind in einem geringeren Abstand von der Oberseite der Frontplatte 118 vorgesehen und liegen ebenfalls diametral einander gegenüber. Des weiteren ist jeweils eine der Verriegelungsnasen 177 mit einer der Verriegelungsnasen 170 axial ausgerichtet.

In einem sich zwischen den Verriegelungsnasen 170 und 177 befindlichen Bereich der Aussenumfangswand des Halses sind, wie es ebenfalls aus Fig. 16 bis 18 hervorgeht, zwei schräg verlaufende Rampen 172 angeformt. Die Rampen 172 liegen symmetrisch zwischen den um 180° gegeneinander versetzten Verriegelungsnasen 170 beziehungsweise 177 und umschlingen auf der zylindrischen Aussenseite des Halses 115 einen Winkelbereich von etwa 90°. Die Rampen 172 haben eine Oberseite, die am einen Ende der Rampen von der Oberseite der Frontplatte 118 ausgeht und am anderen Ende der Rampen in einem vorgegebenen Abstand von der Oberseite der Frontplatte 118 endet. Die beiden einander gegenüberliegenden Rampen 172 haben, in Umfangsrichtung des Halses 115 gesehen, den gleichen Steigungssinn.

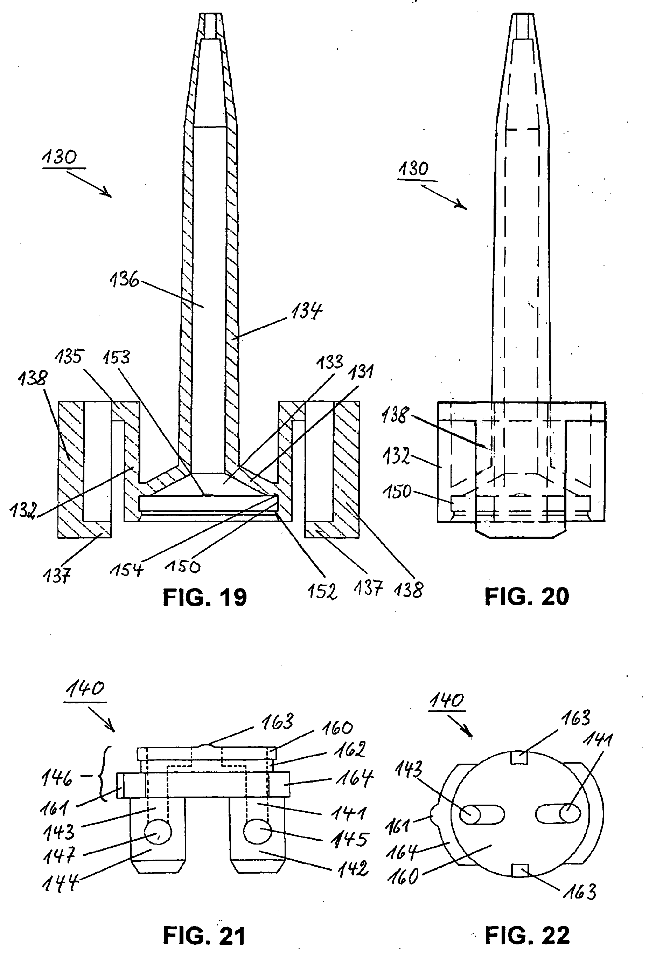

Die Funktion der Verriegelungsnasen 170 und 177 sowie der Rampen 172 wird nachstehend unter Bezugnahme auf den in Fig. 19 und 20 dargestellten Adapter 130 des zweiten Ausführungsbeispiels der Erfindung beschrieben.

Der Adapter 130 unterscheidet sich von dem Adapter 30 des ersten Ausführungsbeispiels im Wesentlichen lediglich dadurch, dass die Verriegelungsarme 138 nicht so weit wie die Verriegelungsarme 38 des ersten Ausführungsbeispieles nach unten oder hinten ragen. Die Verriegelungsarme 138 enden vielmehr kurz oder unmittelbar hinter einem Verriegelungssteg 137, der dem Verriegelungssteg 37 des Adapters 30 des ersten Ausführungsbeispiels entspricht.

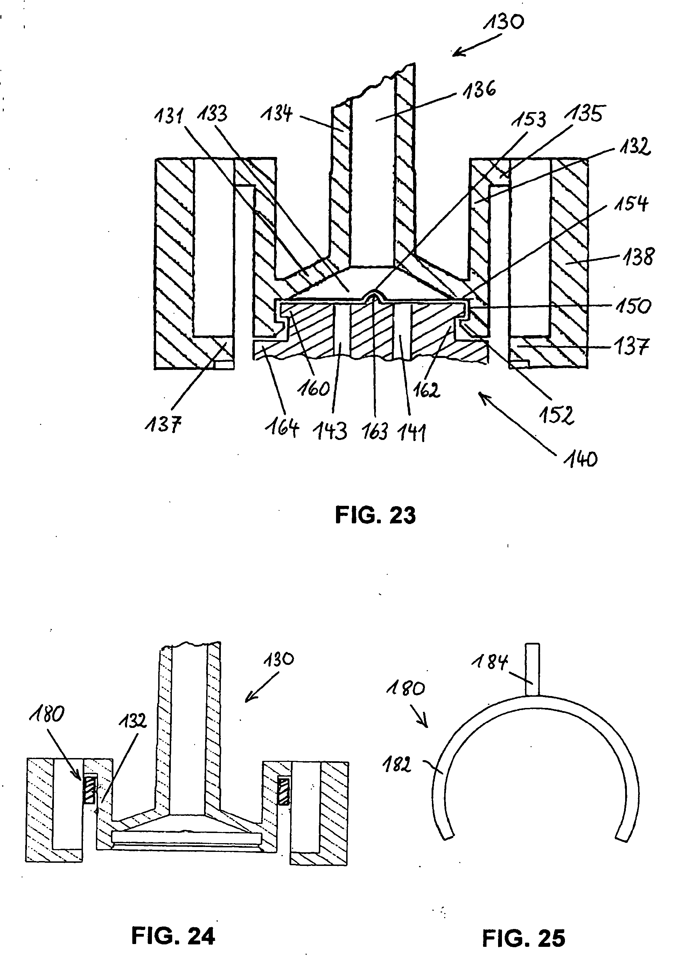

Im übrigen ist wie beim ersten Ausführungsbeispiel der Erfindung, der Adapter 130 mit einem in Fig. 21 und 22 dargestellte Mehrstopfenverschluss 140 drehbeweglich verbunden. Die drehbewegliche Verbindung zwischen dem Adapter 130 und dem Mehrstopfenverschluss 140 ist im einzelnen in Fig. 23 dargestellt, die einen hinteren Abschnitt 132 des Adapters 130 und einen vorderen Abschnitt des Mehrstopfenverschlusses 140 jeweils im Schnitt darstellt. Der Mehrstopfenverschluss 140 hat eine ringförmige Kopfplatte 160, die in eine ringförmige Halterungsnut 150 des Adapters 130 eingeschnappt ist. Die Halterungsnut 150 des Adapters 130 entspricht der Halterungsnut 50 des Adapters 30 des ersten Ausführungsbeispiels und ist, in Axialrichtung gesehen, am hinteren Ende von einer Schulter 152 und am vorderen Ende von einer Schulter 154 begrenzt. Die Schultern 152 und 154 dienen als Anlagefläche für die Kopfplatte 160.

Auf der Oberseite der Kopfplatte 160 sind an diametral einander gegenüberliegenden Stellen zwei in Axialrichtung vorspringende Rastnasen 163 angeformt, die die Funktion der Querrippe 63 der Kopfplatte 60 haben und mit zwei diametral einander gegenüberliegenden Aussparungen 153 in der Schulter 154 derart zusammenwirken, dass der am Adapter 130 drehbar gehalterte Mehrstopfenverschluss 140 in einer vorgegebenen Winkel- oder Drehstellung lösbar verrastet werden kann. Im übrigen hat der Mehrstopfenverschluss 140, ebenso wie der Mehrstopfenverschluss 40, zwei Stopfen 142 und 144, die allerdings einen gleichen Querschnitt haben, einen plattenförmigen Körper 146 bestehend aus der Kopfplatte 160, einer Zwischenplatte 162 und einer Grundplatte 164, sowie zwei Längskanäle 141 und 143 und zwei Querkanäle 145 und 147. An die Umfangswand der Grundplatte 164 ist auch ein radial vorspringender Ansatz 161 angeformt, der mit einer in der Innenfläche des Halses 115 ausgebildeten Längsnut 171 in einer solchen Weise zusammenarbeitet, wie es beim ersten Ausführungsbeispiel beschrieben worden ist.

Nachstehend werden der Zusammenbau und der Betrieb der Mehrkammerampulle gemäss dem zweiten Ausführungsbeispiel erläutert.

Wie beim ersten Ausführungsbeispiel werden vor dem Anbringen der aus dem Adapter 130 und dem Mehrstopfenverschluss 140 bestehenden Baueinheit am Behälter 110 der Adapter 130 und der Mehrstopfenverschluss 140 in einer vorgegebenen Drehstellung miteinander verrastet, in der die Rastnasen 163 in die Rastausnehmungen 153 eingreifen. In diesem verrasteten Zustand wir die Baueinheit 130, 140 von oben her in den Hals 115 des Behälters 110 eingeschoben. Hierbei ist der Ansatz 161 am Mehrstopfenverschluss 140 mit der Längsnut 171 im Hals 115 ausgerichtet. In dieser Drehstellung der Baueinheit 130, 140 gegenüber dem Behälter 110 sind die Verriegelungsarme 138 mit den Verriegelungsnasen 170 ausgerichtet. Beim Einschieben der Baueinheit 130, 140 gelangen deshalb die Verriegelungsstege 137 auf einen oberen abgeschrägten Abschnitt der Verriegelungsnasen 170, und die Stopfen 142 und 144 beginnen in die Austrittsöffnungen 111 und 113 der Kammern 112 und 114 einzutauchen. Wird in dieser Einschubstellung der Adapter 130 weiter in Richtung auf den Behälter 110 gedrückt, schnappen die Verriegelungsstege 137 über die Verriegelungsnasen 170. Dadurch wird eine Rückverschiebung der Baueinheit 130, 140 nach oben oder vorne aus dem Hals 115 des Behälters 110 heraus blockiert. In diesem Blockier- oder Verriegelungszustand sind die Stopfen 142 und 144 gerade soweit in die Austrittsöffnungen 111 und 113 eingetreten, dass die Stopfen die Austrittsöffnungen fluiddicht verschliessen.

Beim Erreichen der oben beschriebenen Verschlussstellung der Stopfen, in der die Verriegelungsstege 137 gerade über die Verriegelungsnasen 170 geschnappt sind, sind die Stirnflächen am unteren Ende der Verriegelungsarme 138 von der Oberseite der Frontplatte 118 beabstandet. Dies ist deswegen der Fall, weil beim zweiten Ausführungsbeispiel der Erfindung die Verriegelungsarme 138 kürzer als die Verriegelungsarme 38 des ersten Ausführungsbeispiels sind. An einer geeigneten Stelle zwischen dem Adapter 130 und dem Behälter 110 ist jedoch beim zweiten Ausführungsbeispiel vorzugsweise ein nicht dargestelltes Anschlagstück vorgesehen, das eine Weiterverschiebung der Baueinheit 130, 140 nach unten oder hinten in Richtung auf den Behälter 110 über die Verschlussstellung des Stopfen 142 und 144 hinaus verhindert.

Ein solches Anschlagstück kann beispielsweise ein an den Adapter 130 oder den Behälter 110 angeformter Clip sein, der mit der Hand abreissbar ist. Der Clip könnte beispielsweise an der Unterseite des Adapters 130 angeformt sein und bei Erreichen der oben beschriebenen Verschlussstellung der Stopfen 142 und 144 an der Oberseite der Frontplatte 118 des Behälters 110 anschlagen.

Wie beim ersten Ausführungsbeispiel der Erfindung können in der Verschlussstellung der Stopfen 142 und 144 die Kammern 112 und 114 von ihrem offenen hinteren Ende her mit Substanzen gefüllt und dann mit Kolben verschlossen werden.

Zum Aktivieren der mit den Substanzen gefüllten Zweikammerampulle gemäss dem zweiten Ausführungsbeispiel wird das oben erwähnte Anschlagstück entfernt und dann die Adapter-Mehrstopfenverschluss-Baueinheit 130, 140 weiter nach hinten in Richtung auf den Behälter 110 geschoben. Im Zuge dieser Verschiebung schnappen die Verriegelungsstege 137 über die Verriegelungsnasen 177 und gleichzeitig gelangen die Stopfen 142 und 144 in die Durchlassstellung. Die über die Verriegelungsnasen 177 geschnappten Verriegelungsstege 137 verhindern, dass in der Durchlassstellung der Stopfen die Baueinheit 130, 140 nach vorne oder oben vom Behälter 110 weggedrückt werden kann. Eine Weiterverschiebung der Baueinheit 130, 140 nach hinten oder unten wird dadurch verhindert, dass die Stirnflächen am unteren Ende der Verriegelungsarme 138 an die Oberseite der Frontplatte 118 anschlagen.

Bezüglich des oben erwähnten Anschlagstücks zeigen Fig. 24 und 25 eine Ausführungsform eines auf den zylindrischen hinteren Abschnitt 132 des Adapters 130 aufschiebbaren Clip 180. Der Clip 180 besteht aus einem offenen ringförmigen Abschnitt 182, der sich beispielsweise über 270° erstreckt, und einem Abziehabschnitt 184, der diametral gegenüber der Öffnung im ringförmigen Abschnitt 182 in der gezeigten Weise an der Aussenseite des ringförmigen Abschnitts 182 angeformt ist. Im aufgeschobenen Zustand schlägt der Clip 180 an der Oberseite des Halses 115 des Behälters 110 an und verhindert auf diese Weise, dass der Adapter 130 über den Verschlusszustand hinaus in den aktivierten Zustand der Mehrkammerampulle geschoben werden kann. Vor dem Aktivieren der Mehrkammerampulle wird der Clip 180 durch Angreifen am Abziehabschnitt 184 mit der Hand vom Hals 132 abgezogen.

Die Zweikammerampulle gemäss dem zweiten Ausführungsbeispiel der Erfindung befindet sich jetzt im aktivierten Zustand, in welchem durch Ausüben von Druck auf die in die Kammern 112 und 114 eingesetzten Kolben die in den Kammern enthaltenen Substanzen durch die Quer- und Längskanäle des Mehrstopfenverschlusses 140 in die Mischkammer 133 des Adapters 130 gedrückt und im gemischten Zustand durch die vordere Spitze des Adapters 130 ausgegeben werden können.

Die Zweikammerampulle gemäss dem zweiten Ausführungsbeispiel ist gleichermassen wie die Zweikammerampulle 100 gemäss dem ersten Ausführungsbeispiel wahlweise zum einmaligen oder mehrmaligen Gebrauch gedacht. Beim mehrmaligen Gebrauch wird bei einer Anwendung lediglich ein Bruchteil der in den Kammern 112 und 114 aufbewahrten Substanzen ausgebracht. Nach Beendigung einer Anwendung wird der Adapter 130 gegenüber dem Behälter 110 aus seiner ursprünglichen Einschubstellung herausgedreht. Bei dieser Drehung des Adapters 130 springen die Verriegelungsarme 138 seitlich aus den Verriegelungsnasen 177 heraus, und die Stirnflächen am unteren Ende der Verriegelungsarme 138 gelangen auf die schräg nach oben oder vorne verlaufenden Rampen 172.

Im Zuge der Weiterdrehung des Adapters 130 gegenüber dem Behälter 110 wird der Adapter 130 durch die Rampen 172 nach oben oder vorne vom Behälter 110 weggedrückt. Hierbei wird auch der mit dem Adapter 110 drehbeweglich verbundene Mehrstopfenverschluss 140 nach vorne oder oben gezogen. Die Steigung der Rampen 172 ist derart bemessen, dass vor dem Erreichen des vordersten Endes der Rampen die Stopfen 142 und 144 praktisch vollständig aus den Austrittsöffnungen 111 und 113 herausgezogen worden sind. Die Baueinheit 130, 140 kann dann ohne Mühe aus dem Hals 115 des Behälters 110 genommen werden. Anschliessend kann eine neue Baueinheit 130, 140 am Behälter 110 angebracht werden.

Es sei erwähnt, dass die Mehrkammerampulle nach der Erfindung direkt mit der Hand unter Verwendung einer in Fig. 7 und 8 dargestellten Kolbeneinheit angewendet oder aber in ein an sich bekanntes Ausbringgerät eingesetzt werden kann. Bei Anwendung in einem Ausbringgerät entfallen die in Fig. 7 und 8 dargestellten Kolbenstangen einschliesslich der Drückerplatte. In die Behälterkammern werden dann lediglich Kolben in Form von Stopfen eingesetzt, die vom Ausbringgerät direkt betätigt werden.

Damit die Einschnappverriegelungen zwischen den Verriegelungsarmen 38, 138 des Adapters 30, 130 und den Verriegelungsnasen 70, 77, 170, 177 sowie die lösbare Verrastung zwischen dem Raststeg 63 oder Rastnasen 163 und den Rastausnehmungen 53, 153 vorgenommen werden können, müssen die dabei zusammenwirkenden Abschnitte der Bauteile aus einem Werkstoff hergestellt sein, der eine gewisse Elastizität zulässt. Zum Herstellen der Teile der erfindungsgemässen Mehrkammerampulle kommen vorzugsweise durch Formpressen oder Spritzgiessen verarbeitbare Thermoplaste in Betracht, wie beispielsweise Polyethylen, Polyethylenterephthalat, Polypropylen, Cycloolefin-Copolymer und dergleichen.

Die oben beschriebenen Ausführungsbeispiele sind nicht als Begrenzung der Erfindung anzusehen. Vielmehr können vom Fachmann Veränderungen und Abwandlungen vorgenommen werden, ohne den Rahmen der Erfindung zu verlassen. So können die Rampen einen anderen Winkelbereich als 90° umschlingen. Auch kann beispielsweise anstelle der Rampe am Hals des Behälters eine Schräge am Adapter oder sowohl am Adapter als auch am Behälter angeformt sein. Gleichermassen können die aufgezeigten Rast- und Verriegelungsmittel in mannigfacher Weise unter Ausübung der gleichen Funktion variiert werden.

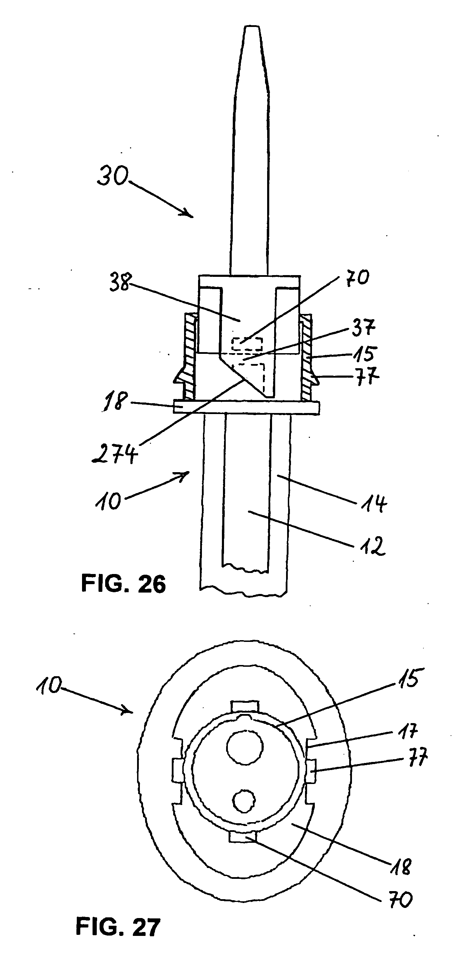

Bezüglich der oben angesprochenen Schrägen am Adapter und/oder Behälter wird auf Fig. 26 bis 33 verwiesen. In Fig. 26 bis 33 werden für im wesentlichen gleiche Teile die gleichen Bezugszahlen wie in Fig. 1 bis 15 verwendet. Abgewandelte oder zusätzliche Merkmale sind mit zusätzlichen Bezugszahlen belegt. Ferner sind in Fig. 26 bis 33 der besseren Übersicht halber lediglich der Behälter 10 und der Adapter 30 dargestellt.

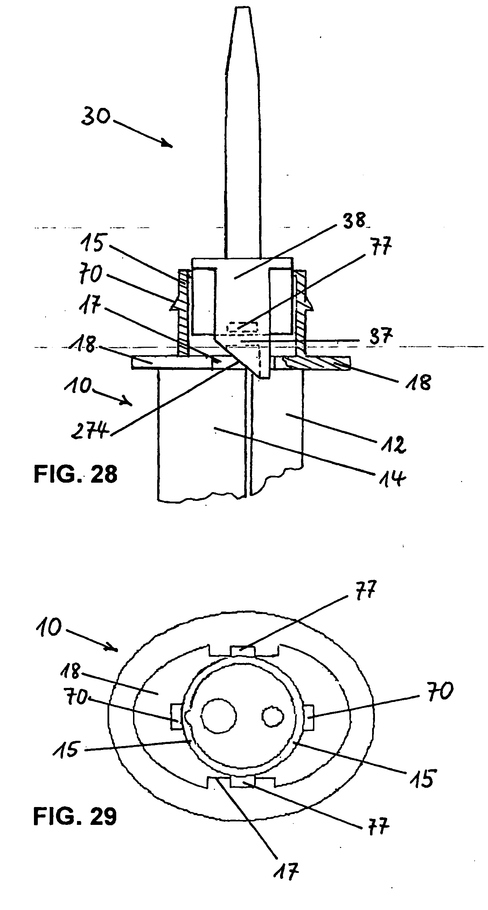

Fig. 26 bis 29 dienen zur Erläuterung einer ersten Weiterbildung des ersten Ausführungsbeispiels der Erfindung. Wie aus Fig. 26 und 28 ersichtlich, ist am unteren oder hinteren Ende des Rastarms 38 eine Schräge 274 ausgebildet. Wie aus Fig. 27 und 29 ersichtlich, ist in der Frontplatte 18 des Behälters 10 die Aussparung 19 weggelassen.

Fig. 26 zeigt die Lage zwischen dem Adapter 30 und dem Behälter 10 im verschlossenen Zustand. Hierbei ist der Verriegelungssteg 37 hinter der Rastnase 70 eingeschnappt und das nicht von der Schräge 274 betroffene unterste oder hinterste Ende des Rastarms 38 schlägt an der Frontplatte 18 des Behälters an, um eine Verschiebung in den aktivierten Zustand zu verhindern.

Zum Aktivieren wird der Adapter 30, wie beim ersten Ausführungsbeispiel der Erfindung, um 90° gedreht und dann nach unten oder hinten verschoben, bis der Verriegelungssteg 37 über die Verriegelungsnase 77 schnappt.

Zur Herausnahme des Adapters 30 und des nicht dargestellten Mehrstopfenverschlusses 40 wird der Adapter 30 im gleichen Richtungssinn weitergedreht. Aufgrund des in Fig. 28 zu sehenden Abstands zwischen der Schräge 274 und dem Rand der Ausnehmung 17 gelangt bei dieser Weiterdrehung der Adapter 30 zunächst in den entriegelten Zustand. Anschliessend greift die Schräge 274 am Rand der Ausnehmung 17 an und drückt den Adapter 30 zusammen mit dem nicht dargestellten Mehrstopfenverschluss 40 nach oben aus dem Hals 15 des Behälters 10 heraus.

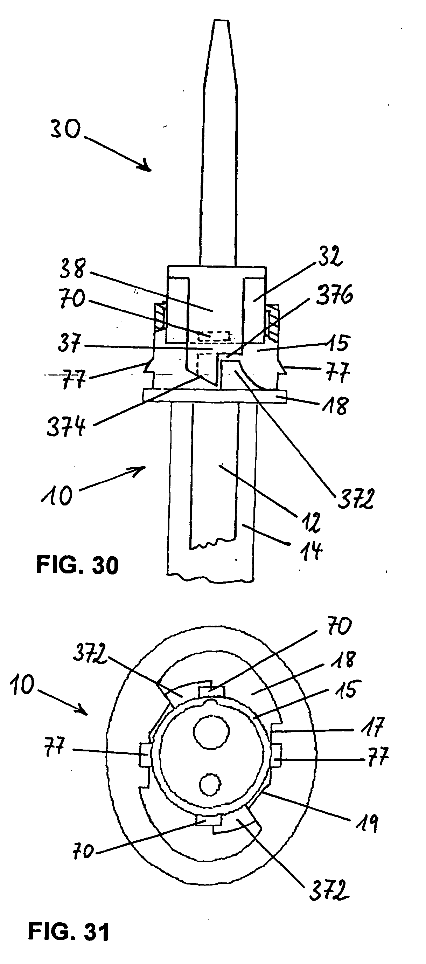

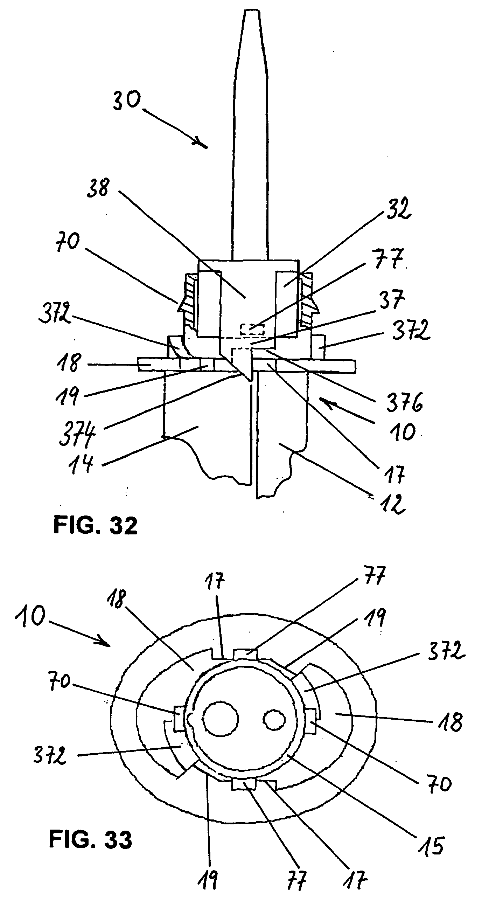

Fig. 30 bis 33 dienen zur Erläuterung einer zweiten Weiterbildung des ersten Ausführungsbeispiels der Erfindung. Wie aus Fig. 30 und 32 ersichtlich, ist in der in der Darstellung rechten Hälfte des unteren Abschnitts des Verriegelungsarms 38 eine rechteckförmige Aussparung 376 vorgesehen, die höchstens bis zur Unterseite des Verriegelungsstegs 37 reicht. Die in der Darstellung linke Hälfte des unteren Abschnitts des Verriegelungsstegs 38 ist in der gezeigten Weise in Form einer Schräge 374 ausgebildet.

Wie aus Fig. 30 bis 33 ersichtlich, sind auf der Oberseite der Frontplatte 18 zwei diametral einander gegenüberliegende Rampen 372 angeformt.

Fig. 30 zeigt die Lage zwischen dem Behälter 10 und dem Adapter 30 im verschlossenen Zustand der Mehrkammerampulle. Hierbei ist der Verriegelungssteg 37 hinter der Verriegelungsnase 70 eingeschnappt. Eine Verschiebung des Adapters 30 nach unten oder hinten über den verschlossenen Zustand hinaus ist dadurch verhindert, dass das unterste oder hinterste Ende der Schräge 374 an der Oberseite der Frontplatte 18 und/oder die obere Kante der Ausnehmung 376 auf der Oberseite der Rampe 372 anschlägt. Die Rampe 372 ist derart ausgebildet, dass sie bei der Verriegelungsnase 70 ihren grössten Abstand von der Oberseite der Frontplatte 18 hat und von dort in Richtung auf die Ausnehmung 19 bis hin zum Niveau der Frontplatte 18 abfällt.

Zum Aktivieren der Mehrkammerampulle wird, wie bei der ersten Weiterbildung des ersten Ausführungsbeispiels der Erfindung, der Adapter 30 um 90° gegenüber dem Behälter gedreht und dann nach unten in die in Fig. 32 gezeigte Stellung geschoben. In diesem Zusammenhang ist zu erwähnen, dass bei der Drehung aus dem in Fig. 30 dargestellten Verschlusszustand das bei der Verriegelungsnase 70 befindliche Ende einen Anschlag darstellt, der eine Drehung in nur einer Richtung zulässt. Bei dem dargestellten Beispiel handelt es sich hierbei um eine Rechtsdrehung.

Zur Herausnahme des Adapters 30 einschliesslich des nicht dargestellten Mehrstopfenverschlusses 40 wird der Adapter 30 aus der in Fig. 32 dargestellten Stellung weiter nach rechts gedreht. Aufgrund des Abstands zwischen dem Rand der Ausnehmung 19 und der Schräge 374 gelangt der Adapter zunächst in den entriegelten Zustand. Anschliessend läuft bei einer Weiterdrehung nach rechts die Schräge 374 am Rand der Ausnehmung 19 hoch und gelangt auf die Oberseite der Rampe 372. Die Abmessungen der Rampe 372 und der Schräge 374 sind derart getroffen, dass bei der Weiterdrehung des Adapters 30 aus dem aktivierten Zustand heraus der Verriegelungsarm 38 soweit nach oben geschoben ist, dass vor Erreichen der Verriegelungsnase 70 der Verriegelungssteg 37 nach oben über die Verriegelungsnase 70 angehoben ist, so dass eine die Herausnahme des Adapters 30 störende erneute Verriegelung mit Sicherheit vermieden ist.

## Patentansprüche

1. Mehrkammerampulle zum Ausgeben eines aus mehreren Substanzen bestehenden Gemisches, aufweisend:
einen Behälter (10; 110) mit mehreren Kammern (12,14; 112,114), die parallel zueinander angeordnet sind und von denen jede ein offenes hinteres Ende zum Eingeben einer der Substanzen und anschliessenden Einsetzen eines Kolbens (21,23) in die Kammer und ein mit einer Austrittsöffnung (11,13; 111,113) versehenes vorderes Ende aufweist, und mit einem an die vorderen Enden der Kammern angeformten hohlzylindrischen Hals (15; 115), in den die Austrittsöffnungen der Kammern münden,
einen in den Hals (15; 115) einsetzbaren Mehrstopfenverschluss (40; 140) mit einem plattenförmigen Körper (46; 146) und mit mehreren, an einer Unterseite des Körpers angeformten Stopfen (42,44; 142,144), die von der Unterseite des Körpers aus nach unten ragen und beim Einsetzen des Mehrstopfenverschlusses (40; 140) in den Hals (15; 115) in die Austrittsöffnungen (11,13; 111,113) der Kammern eintreten, wobei der Mehrstopfenverschluss derart ausgebildet ist, dass die Stopfen in einer ersten Eintrittsstellung eine Verschlussstellung einnehmen, in der die Austrittsöffnungen fluiddicht verschlossen sind, und in einer weiter in den Hals (15; 115) des Behälters (10; 110) verschobenen zweiten Eintrittsstellung eine Durchlassstellung einnehmen, in der ein Fluiddurchtritt durch die Austrittsöffnungen gestattet ist,
einen Adapter (30; 130) mit einem den Adapter durchsetzenden Ausgabekanal (36; 136) und mit einem zum hohlzylindrischen Hals (15; 115) passenden zylindrischen hinteren Abschnitt (32; 132), der im Anschluss an den Mehrstopfenverschluss fluiddicht in den Hals einsetzbar ist und darin im eingesetzten Zustand verschiebbar und drehbar ist, wobei durch ein Verschieben des Adapters in Richtung des Behälters (10; 110) die Stopfen von der Verschlussstellung in die Durchlassstellung schiebbar sind, und
am Behälter (10; 110) und Adapter (30; 130) angeformte Verriegelungsmittel (37,77; 137,177), die derart zusammenwirken, dass sie den Adapter beim Erreichen der durch den Adapter bewirkten Verschiebung der Stopfen (42,44; 142,144) von der Verschlussstellung in die Durchlassstellung gegen eine Rückverschiebung nach vorne verriegeln, **dadurch gekennzeichnet,**
**dass** der plattenförmige Körper (46; 146) des Mehrstopfenverschlusses (40; 140) am hinteren Abschnitt (32; 132) des Adapters (30; 130) drehbar gehaltert ist und die an dem Behälter (10; 110) und dem Adapter (30; 130) angeformten Verriegelungsmittel (37,77; 137, 177) derart ausgebildet sind, dass der Adapter relativ zu dem in den Austrittsöffnungen der Kammern nicht verdrehbar gehaltenen Mehrstopfenverschluss in eine entriegelte Stellung drehbar ist, in der der Adapter (30; 130) nicht mehr verriegelt ist und zusammen mit dem Mehrstopfenverschluss (40; 140) aus dem Hals (15; 115) des Behälters (10; 110) herausnehmbar und vom Behälter trennbar ist.

2. Mehrkammerampulle nach Anspruch 1, bei der am Behälter und/oder am Adapter eine Schräge (172; 274; 372; 374) angeformt ist, die im Verlauf der Drehung des Adapters (30; 130) in die entriegelte Stellung den Adapter zusammen mit dem Mehrstopfenverschluss (40; 140) vom Behälter (10; 110) wegzieht.

3. Mehrkammerampulle nach Anspruch 2, bei der am Hals (15; 115) des Behälters (10; 110) eine Rampe (172; 372) angeformt ist.

4. Mehrkammerampulle nach einem der vorstehenden Ansprüche, bei der zwischen dem Behälter (10; 110) und Adapter (30; 130) Anschlagmittel vorgesehen sind, die derart zusammenwirken, dass sie bei einem anfänglichen Einsetzen des Adapters in den Behälter eine durch den Adapter bewirkte Verschiebung des Mehrstopfenverschlusses (40; 140) nach hinten über die Verschlussstellung der Stopfen (42,44; 142,144) hinaus begrenzen.

5. Mehrkammerampulle nach Anspruch 4, bei der die Anschlagmittel ein am Behälter und/oder Adapter angeformtes, entfernbares Anschlagstück aufweisen, das nach seiner Entfernung eine Weiterverschiebung des Adapters (130) nach hinten in Richtung auf den Behälter (110) von der Verschlussstellung in die Durchlassstellung der Stopfen (142, 144) zulässt.

6. Mehrkammerampulle nach einem der vorstehenden Ansprüche, bei der am Adapter (30; 130) und Behälter (10; 110) weitere Verriegelungsmittel (37, 70; 137,170) angeformt sind, die derart zusammenwirken, dass sie bei einem anfänglichen Einsetzen des Adapters in den Behälter den Adapter beim Erreichen der Verschlussstellung der Stopfen gegen eine Rückverschiebung nach vorne verriegeln.

7. Mehrkammerampulle nach einem der vorstehenden Ansprüche, bei der der Behälter (10; 110) eine sich quer zur Längsachse der Kammern (12,14; 112, 114) erstreckende Frontplatte (18; 118) hat, die mit einer unteren Plattenfläche an die vorderen Enden der Kammern (12,14; 112, 114) angeformt ist und in der die Austrittsöffnungen (11,13; 111, 113) der Kammern ausgebildet sind, und der Behälterhals (15; 115) ein offenes vorderes Ende und ein an eine obere Plattenfläche der Frontplatte (18; 118) angeformtes hinteres Ende aufweist, das die in der Frontplatte (18; 118) ausgebildeten Austrittsöffnungen (11,13; 111, 113) der Kammern umrandet.

8. Mehrkammerampulle nach einem der vorstehenden Ansprüche, bei der am Adapter (30; 130) ein Querträger (35; 135) angeformt ist, an den nach hinten ragende Verriegelungsarme (38; 138) angeformt sind, die, wenn der hintere Abschnitt (32; 132) des Adapters in den Hals (15; 115) des Behälters eingesetzt ist, parallel zur Aussenumfangswand des Behälterhalses nach hinten verlaufen, und bei der an der Aussenumfangswand des Behälterhals (15; 115) Verriegelungsnasen (70, 77; 170,177) angeformt sind, die mit den Verriegelungsarmen (38; 138) zusammenwirken.

9. Mehrkammerampulle nach Anspruch 3 und 8, bei der im Verlauf der Drehung des Adapters (130) in die entriegelte Stellung die unteren Stirnflächen der Verriegelungsarme (138) auf der Rampe (172) laufen.

10. Mehrkammerampulle nach Anspruch 7 und 8, bei der sich die Frontplatte(18) des Behälters (10) in Radialrichtung über eine Aussenumfangswand des Behälterhalses (15) hinaus erstreckt und bei der beim anfänglichen Einsetzen des Adapters (30) in den Behälter (10) in einer ersten Drehstellung zwischen Adapter und Behälter beim Erreichen einer durch den Adapter bewirkten Verschiebung des Mehrstopfenverschlusses in die Verschlussstellung die Verriegelungsarme (38) an der oberen Plattenfläche der Frontplatte (18) anschlagen.

11. Mehrkammerampulle nach Anspruch 10, bei der der Adapter (30) aus der ersten Drehstellung heraus in eine zweite Drehstellung drehbar ist, in der die Verriegelungsarme (38) des Adapters mit Aussparungen (17) in der Frontplatte (18) ausgerichtet sind, so dass in der zweiten Drehstellung der Adapter weiter nach hinten geschoben werden kann, in der die Verriegelungsarme (38) nach hinten über die Frontplatte (18) des Behälters hinausragen und den Adapter beim Erreichen einer durch den Adapter (30) bewirkten Verschiebung des Mehrstopfenverschlusses (40) in die Durchlassstellung der Stopfen (42,44) gegen eine Rückverschiebung nach vorne verriegeln.

12. Mehrkammerampulle nach Anspruch 11, bei der die Aussparungen (17,19) in der Frontplatte (18) sich über einen Winkelbereich von der zweiten Drehstellung bis in eine verriegelungsfreie dritte Stellung erstrecken, so dass der in der zweiten Drehstellung verriegelte Adapter (30) aus der Verriegelung heraus in die dritte Drehstellung gedreht werden kann, in der der Adapter (30) zusammen mit dem Mehrstopfenverschluss (40) nach vorne aus dem Behälterhals herausgezogen werden kann.

13. Mehrkammerampulle nach einem der vorstehenden Ansprüche, bei der am hinteren Abschnitt des Adapters (30; 130) und am plattenförmigen Körper (46; 146) des Mehrstopfenverschlusses (40; 140) Rastmittel (53,63; 153,163) ausgebildet sind, die derart zusammenwirken, dass der Adapter (30; 130) und der Mehrstopfenverschluss (40; 140) in einer vorgegebenen Drehwinkelstellung miteinander verrastbar sind.

14. Mehrkammerampulle nach einem der vorstehenden Ansprüche, bei der an den plattenförmigen Körper (46; 146) des Mehrstopfenverschlusses ein in Querrichtung vorspringenden Ansatz (61; 161) angeformt ist, der mit einer im Inneren des Halses (15; 115) ausgebildeten Längsnut (71; 171) so zusammenwirkt, dass der Mehrstopfenverschluss nur in einer vorgegebenen Drehwinkelstellung in den Behälterhals (15; 115) eingesetzt werden kann, in der die Stopfen (42,44; 142,144)) des Mehrstopfenverschlusses mit jeweils zugeordneten Austrittsöffnungen (11,13; 111,113) der Kammern ausgerichtet sind.

15. Mehrkammerampulle nach einem der vorstehenden Ansprüche, bei der die Kammern (12,14) voneinander abweichende Querschnittsabmessungen haben und in einer dazu passenden Weise die Kolben (21,23), die Austrittsöffnungen (11,13) und die Stopfen (42,44) dimensioniert sind.

## Claims

1. Multi-chamber ampoule for the dispensing of a mixture made of one or more substances and equipped with:
a box (10; 110) made of several chambers (12; 14; 11, 114) with a parallel alignment one to another, wherein each of them is equipped with an open back ending for the dispensing of one of the substances and for the subsequent application of a flask (21, 23) inside the chamber and
with a frontal ending equipped with an exit opening (11, 13; 11, 113) and with a neck having the shape of a hollow cylinder at the frontal endings of the chambers (15; 115) into which the exit openings of the chamber empty,
a multi-tamping stopper (40; 140) equipped with a platform-shaped body (46; 146) to be inserted into the neck (15; 115) and with several tamping elements (42, 44; 142, 144) adjusted on the bottom side of the body and protruding towards the bottom side and entering the neck (15; 115) into the exit openings (11, 13; 111, 113) as soon as the multi-tamping stopper (40; 140) is inserted, wherein the multi-tamping stopper is shaped so that the tamping elements in their first entering position take a locking position in which the exit openings are impermeably closed, and in a second entering position moved into the neck (15; 115) of the box (10; 110) take an outlet position in which the fluid outlet is permitted through the exit openings,
an adapter (30; 130) equipped with an outlet channel (36; 136) interspersed with an adapter and with a rear cylindrical section (32; 132) fitting the hollow cylindrical neck (15; 115), which can be inserted into the neck in a permeable connection with the multi-tamping stopper and which can be moved and rotated in this inserted position, wherein by moving the adapter towards the direction of the box (10; 110) the tamping elements can be moved from the locking position into the outlet position, and
blocking means (37, 77; 137, 177) adjusted on the box (10; 110) and on the adapter (30; 130) which interact so that they close the adapter forwards after the displacement of the tamping elements (42, 44; 142, 144) from the locking position into the outlet position to prevent from back displacement, **characterized in that**
the platform-shaped body (46; 146) of the multi-tamping stopper (40; 140) on the rear section (32; 132) of the adapter (30; 130) is pivoted and the blocking means (37, 77; 137, 177) adjusted on the box (10; 110) and on the adapter (30; 130) are designed so that the adapter can be rotated into an unlocked position relatively to the multi-tamping stopper having a nonrotating position within the exit openings of the chambers, wherein in this position the adapter is not locked anymore and can be extracted from the neck (15; 115) of the box (10; 110) together with the multi-tamping stopper (40; 140) and as a consequence it can be separated from the box.

2. Multi-chamber ampoule according to claim 1, wherein on the box and/or on the adapter a chamfer (172; 274; 372; 374) is adjusted which moves the adapter together with the multi-tamping stopper (40; 140) from the box (10; 110) during the rotation of the adapter (30; 130) into the unlocked position.

3. Multi-chamber ampoule according to claim 2, wherein on the neck (15; 115) of the box (10; 110) a ramp (172; 372) is adjusted.

4. Multi-chamber ampoule according to one of the previous claims, wherein between the box (10; 110) and the adapter (30; 130) there are separate lifting accessories which interact one with another so that in case of an initial insertion of the adapter into the box they limit the displacement of the multi-tamping stopper (40; 140) backwards beyond the locking position of the stopper (42; 44; 142, 144).

5. Multi-chamber ampoule according to claim 4, wherein the separate lifting accessories are equipped with a removable lifting element adjusted on the box and/or on the adapter permitting an additional adjustment of the adapter (130) backwards towards the direction of the box (110) from the locking position into the outlet position of the stopper (142, 144) after its removal.

6. Multi-chamber ampoule according to one of the previous claims, wherein on the adapter (30; 130) and on the box (10; 110) additional closing means (37, 70; 137, 170) are adjusted which interact so that by the initial insertion of the adapter into the box as soon as the locking position of the stopper is reached they close the adapter forwards to prevent from back displacement.

7. Multi-chamber ampoule according to one of the previous claims, wherein the box (10; 110) is equipped with a front platform (18; 118) extending transversally to the longitudinal axle of the chambers (12, 14; 112, 114) and adjusted with a platform surface on the under part of the frontal endings of the chambers (12, 14; 112, 114) and in which the exit openings (11, 13; 11, 113) of the chambers are designed, and wherein the neck of the box (15; 115) is equipped with an open frontal ending and a rear ending adjusted on the upper platform surface of the frontal platform (18; 118) wherein the rear ending borders the exit openings (11, 13; 11, 113) of the chambers designed in the frontal platform (18; 118).

8. Multi-chamber ampoule according to one of the previous claims, wherein on the adapter (30; 130) there is a crossbar (35; 135) adjusted on which protruding towards the rear side there are adjusted locking arms (38; 138) which move backwards in a parallel direction to the external circumference of the box neck as soon as the rear section (32; 132) of the adapter is inserted into the neck (15; 115) of the box, wherein the on the external circumference of the box neck (15; 115) locking nibs (70, 77; 170, 177) interacting with the locking arms (38; 138) are adjusted.

9. Multi-chamber ampoule according to claim 3 and 8, wherein during the rotation of the adapter (130) into the unlocked position the lower front surfaces of the locking arms (138) are moved on the ramp (172).

10. Multi-chamber ampoule according to claim 7 and 8, wherein the frontal platform (18) of the box (10) extends into a radial direction over an external circumference wall of the box neck (15) and wherein at the initial insertion of the adapter (30) into the box (10) during the first rotating position between the adapter and the box as soon as the displacement of the multi-tampering stopper produced by the adapter into the locking position the locking arms (38) attach the upper platform surface of the frontal platform (18).

11. Multi-chamber ampoule according to claim 10, wherein the adapter (30) can be rotated from the first rotating position into the second rotating position, in which the locking arms (38) of the adapter are aligned in the frontal platform with openings (17), so that in the second rotating position the adapter can be displaced more backwards, in which the locking arms (38) protrude backwards over the frontal platform (18) of the box and lock the adapter as soon as the displacement of the multi-tamping stopper (40) into the outlet position of the stopper (42, 44) is produced by the adapter to prevent it from a forward displacement.

12. Multi-chamber ampoule according to claim 11, wherein the openings (17, 19) in the frontal platform (18) extend over the angle area from the second rotating position into a third locking-free position, so that the adapter (30) locked in the second rotating position can be rotated our from the third rotating position, in which the adapter (30) can by pulled forwards together with the multi-tamping stopper (40) out from the box neck.

13. Multi-chamber ampoule according to one of the previous claims, wherein on the rear section of the adapter (30; 130) and on the platform-shaped body (46; 146) of the multi-tamping stopper (40; 140) stopping means (40; 140) are designed which interact so that the adapter (30; 130) and the multi-tamping stopper (40; 140) can be locked one in another in a preset rotation angle position.

14. Multi-chamber ampoule according top one of the previous claims, wherein on the platform-shaped body (46; 146) of the multi-tamping stopper a protruding beginning (61; 161) is transversally adjusted and interacts with the longitudinal slot (71; 171) designed in the internal area of the neck (15; 115) so that the multi-tamping stopper can be used only in a preset rotation angle position inside the box neck (15; 115) in which the stopper (42, 44; 142, 144) of the multi-tamping stopper is respectively aligned with the corresponding exit openings (11, 13; 111, 113).

15. Multi-chamber ampoule according to one of the previous claims, wherein the chambers (12, 14) are equipped with different cross-section measurements and wherein the flasks (21, 23), the exit openings (11, 13) and the stoppers (42, 44) are dimensioned accordingly.

## Revendications

1. Ampoule à compartiments multiples pour la distribution d'un mélange composé de plusieurs substances, présentant :
un réservoir (10; 100) avec plusieurs compartiments (12, 14; 112, 114) qui sont placés parallèlement les uns aux autres et qui présentent chacun une extrémité arrière ouverte pour introduire l' une des substances et pour l'insertion ultérieure d'un piston (21, 23) dans le compartiment et une extrémité à l'avant avec un orifice d'écoulement (11, 13; 111, 113) et avec un collet en forme de cylindre creux (15; 115) formé sur l'extrémité avant des compartiments, dans lequel les orifices d'écoulement des compartiments débouchent,
un obturateur à bouchons multiples (40; 140) insérable dans le collet (15; 115) avec un corps en forme de plaque (46; 146) et avec plusieurs bouchons formés sur une partie inférieure du corps (42, 44; 142, 144) qui dépassent de la partie inférieure du corps vers le bas et lors de l'insertion de l'obturateur à bouchons multiples (40; 140) dans le collet entrent dans les orifices d'écoulement (11,13; 111, 113) des compartiments, l'obturateur à bouchons multiples étant conçu de telle manière que les bouchons dans une première position d'entrée prennent une position de fermeture dans laquelle les orifices d'écoulement sont fermés avec étanchéité aux fluides, et dans une deuxième position d'entrée déportée à nouveau dans le collet (15; 115) du réservoir (10; 110) ils prennent une position de passage, dans laquelle un passage de fluide est rendu possible par les orifices d'écoulement,
un adaptateur (30; 130) avec un canal de sortie (36; 136) traversant l'adaptateur et avec une encoche (32; 132) cylindrique arrière adaptée à un collet en forme de cylindre creux, qui, en connexion avec l'obturateur à bouchons multiples est insérable de manière étanche dans le collet et est mobile et rotatif dans la position insérée, les bouchons pouvant être décalés de la position de fermeture à la position de passage par un déplacement de l'adaptateur en direction du réservoir (10; 110) et des dispositifs de verrouillage (37, 77; 137, 177) formés sur le réservoir (10; 110) et l'adaptateur (30; 130), qui fonctionnent en interaction de telle manière qu'ils verrouillent l'adaptateur en réalisant le décalage des bouchons (42, 44; 142, 144) provoqué par l'adaptateur, de la position de fermeture à la position de passage contre un mouvement de retour vers l'avant, **caractérisé en ce que**
le corps en forme de plaque (46; 146) de l'obturateur à bouchons multiples (40; 140) est supporté en rotation dans l'encoche arrière (32; 132) de l'adaptateur (30; 130) et les dispositifs de verrouillage (37, 77; 137, 177) formés sur le réservoir (10; 110) et
l'adaptateur (30; 130) sont conçus de telle manière que l'adaptateur, par rapport à l'obturateur à bouchons multiples ayant un blocage pour son empêcher son pivotement dans les orifices d'écoulement des compartiments, est pivotant en position débloquée, dans laquelle l'adaptateur (30; 130) n'est plus verrouillé et conjointement avec l'obturateur à bouchons multiples (40; 140) est amovible du collet (15; 115) du réservoir (10; 110) et séparable du réservoir.

2. Ampoule à compartiments multiples selon revendication 1 **caractérisée en ce qu'**une inclinaison (172; 274; 372; 374) formée sur le réservoir et/ou sur l'adaptateur, qui au cours de la rotation de l'adaptateur (30; 130) dans la position débloquée enlève l'adaptateur conjointement avec l'obturateur à bouchons multiples (40; 140) du réservoir (10; 110).

3. Ampoule à compartiments multiples selon revendication 2 **caractérisées en ce qu'**une rampe (172; 372) est formée sur le collet (15, 115) du réservoir (10; 110).

4. Ampoule à compartiments multiples selon l'une des revendications antérieures **caractérisée en ce que** des dispositifs de levage sont prévus entre le réservoir (10; 110) et l'adaptateur (30; 130), qui fonctionnent en interaction de telle manière que lors d'une insertion initiale de l'adaptateur dans le réservoir ils limitent vers l'arrière un déplacement de l'obturateur à bouchons multiples (40; 140), provoqué par l'adaptateur, par la position de fermeture des bouchons (42, 44; 142, 144).

5. Ampoule à compartiments multiples selon revendication 4 **caractérisée en ce que** les dispositifs de levage présentent une pièce de levage extractible formée sur le réservoir et/ou l'adaptateur, qui après son extraction autorise un nouveau déplacement de l'adaptateur (130) vers l'arrière en direction du réservoir (110) de la position de fermeture à la position de passage des bouchons (142, 144).

6. Ampoule à compartiments multiples selon l'une des revendications antérieures **caractérisée en ce que** d'autres dispositifs de verrouillage (37, 70; 137, 170) sont formés sur l'adaptateur (30; 130) et le réservoir (10; 110), qui fonctionnent en interaction de telle manière que lors d'une insertion initiale de l'adaptateur dans le réservoir, ils verrouillent l'adaptateur en atteignant la position de fermeture des bouchons contre un mouvement de retour vers l'avant.

7. Ampoule à compartiments multiples selon l'une des revendications précédentes **caractérisée en ce que** le réservoir (10; 110) a une plaque frontale (18; 118) s'étendant transversalement à l'axe longitudinal des compartiments (12, 14; 112, 114) qui est formée avec une surface de plaque inférieure sur l'extrémité avant des compartiments (12, 14; 112, 114) et dans laquelle les orifices d'écoulement sont formés et le collet du réservoir (15; 115) présente une extrémité avant ouverte et une extrémité arrière formée sur une surface de plaque supérieure de la plaque frontale (18, 118), qui encercle les orifices d'écoulement (11, 13; 111, 113) des compartiments formés sur la plaque frontale (18; 118).

8. Ampoule à compartiments multiples selon l'une des revendications précédentes **caractérisée en ce qu'**une traverse (35; 135) est formée sur l'adaptateur (30; 130) sur lequel sont formés des bras de verrouillage (38; 138) dépassant vers l'arrière lorsque l'encoche arrière (32; 132) de l'adaptateur est insérée dans le collet (15; 115) du réservoir, disposé parallèlement à la paroi périphérique externe du collet du réservoir (15; 115) et **caractérisée en ce que** des nez de verrouillage (70, 77; 170, 177) sont formés sur la paroi périphérique externe du collet du réservoir (15; 115), fonctionnant en interaction avec les bras de verrouillage (38; 138).

9. Ampoule à compartiments multiples selon revendication 3 et 8 **caractérisée en ce qu'**au cours de la rotation de l'adaptateur (130) vers la position de blocage les fronts inférieurs des bras de verrouillage (138) coulissent sur la rampe (172).

10. Ampoule à compartiments multiples selon revendications 7 et 8 **caractérisée en ce que** la plaque frontale (18) du réservoir (10) s'étend en direction radiale sur une paroi périphérique externe du collet du réservoir (15) et **caractérisée en ce que** lors de l'insertion initiale de l'adaptateur (30) dans le réservoir (10) dans une position première position de rotation entre adaptateur et réservoir réalisant un déplacement de l'obturateur à bouchons multiples vers la position de fermeture provoqué par l'adaptateur, les bras de verrouillage sont amenés en butée contre la superficie de plaque de la plaque frontale (18).

11. Ampoule à compartiments multiples selon revendication 10 **caractérisée en ce que** l'adaptateur est rotatif depuis une première position de rotation à une deuxième position de rotation, dans laquelle les bras de verrouillage (38) de l'adaptateur sont alignés avec des évidements (17) sur la plaque frontale (18) de telle manière que dans la deuxième position de rotation l'adaptateur puisse être repoussé vers l'arrière, que les bras de verrouillage (38) dépassent la plaque frontale (18) du réservoir vers l'arrière et verrouillent l'adaptateur lorsqu'est réalisé le déplacement de l'obturateur à bouchons multiples (40) provoqué par l'adaptateur (30) vers la position de passage des bouchons (42, 44) pour éviter un mouvement de retour vers l'avant.

12. Ampoule à compartiments multiples selon revendication 11 **caractérisée en ce que** des évidements (17, 19) dans la plaque frontale (18) s'étendent sur un espace angulaire de la deuxième position de rotation jusqu'à une troisième position sans verrouillage de telle manière que l'adaptateur (30) verrouillé dans la deuxième position de rotation peut se libérer du verrouillage par rotation allant dans la troisième position de rotation, dans laquelle l'adaptateur (30) peut être retiré du collet du réservoir vers l'avant avec l'obturateur à bouchons multiples. (40).

13. Ampoule à compartiment multiple selon l'une des revendications précédentes **caractérisée en ce que** sur l'encoche arrière de l'adaptateur (30; 130) et sur le corps en forme de plaque (46; 146) de l'obturateur à bouchons multiples (40; 140) des quadrillages (53, 63; 153, 163) sont formés fonctionnant en interaction de telle manière que l'adaptateur (30; 130) et l'obturateur à bouchons multiples (40; 140) peuvent s'enclencher l'un avec l'autre dans une position angulaire de rotation.

14. Ampoule à compartiment multiple selon l'une des revendications précédentes **caractérisée en ce que** sur le corps en forme de plaque (46; 146) de l'obturateur à bouchons multiples une embouchure (61; 161) proéminente en direction transversale est formée, qui fonctionne en interaction avec une cannelure formée à l'intérieur du collet (15; 115) de telle manière que l'obturateur à bouchons multiples ne peut être inséré que dans une position angulaire de rotation donnée dans le collet du réservoir (15; 115), dans laquelle les bouchons (42, 44; 142, 144) de l'obturateur à bouchons multiples sont alignés avec les orifices d'écoulement (11, 13; 111, 113) assignés respectivement.

15. Ampoule à compartiments multiples selon l'une des revendications précédentes **caractérisée en ce que** les compartiments (12, 14) ont une dimension de coupe transversale divergente et les pistons (21, 23) les orifices d'écoulement (11, 13) et les bouchons (42, 44) sont dimensionnés d'une manière appropriée.
